# EUROPEAN PATENT APPLICATION

(11) **EP 4 071 479 A1**
(43) Date of publication of application: **12.10.2022**
(21) Application number: 20913385.9
(22) Date of filing: 17.04.2020
(51) Int. Cl.: G01N 33/68, C07K 14/47

(54) **PROTEIN ANTIGEN COMBINATION FOR DETECTING ALZHEIMER'S DISEASE AUTOANTIBODY AND APPLICATION OF PROTEIN ANTIGEN COMBINATION**

(30) Priority: 15.01.2020 CN 202010039229
(71) Applicant: SHANGHAI ZHONGQI BIOTECHNOLOGY CO., LTD, Shanghai 200135 (CN)
(72) Inventor: SHEN, Lu, Shanghai 200135 (CN); WU, Qihui, Shanghai 200135 (CN); YIN, Kun, Shanghai 200135 (CN); ZOU, Yonghong, Shanghai 200135 (CN)
(74) Representative: Elzaburu S.L.P.
(86) International application number: PCT/CN2020/085224
(87) International publication number: WO 2021/142963

(57) **Abstract**

A protein antigen combination for detecting Alzheimer's disease autoantibodies in human serum sample and an application thereof are disclosed. The protein antigen combination includes at least two protein fragments selected from the following proteins: MAPT, ADARB1, P21, DNAJC8, RAGE, ASXL1, and JMJD2D.Based on the protein antigen composition, a kit for diagnosis, especially for early diagnosis of Alzheimer's disease or its related risks, can be prepared. By using the protein antigen composition provided by the invention, early Alzheimer's disease can be quickly and accurately diagnosed, which has important practical significance.

<110>   Shanghai Zhongqi Biotechnology CO., LTD.
<120>   Protein antigen combination, detection method and testing kit using
the same
<160>   9
<210>   1
<211>   257
<212>   PRT
<213>   MAPT[502-758]
<400>   1
Ser Gly Glu Pro Pro Lys Ser Gly Asp Arg Ser Gly Tyr Ser Ser Pro
1               5                   10                  15
Gly Ser Pro Gly Thr Pro Gly Ser Arg Ser Arg Thr Pro Ser Leu Pro
            20                  25                  30
Thr Pro Pro Thr Arg Glu Pro Lys Lys Val Ala Val Val Arg Thr Pro
        35                  40                  45
Pro Lys Ser Pro Ser Ser Ala Lys Ser Arg Leu Gln Thr Ala Pro Val
    50                  55                  60
Pro Met Pro Asp Leu Lys Asn Val Lys Ser Lys Ile Gly Ser Thr Glu
65                  70                  75                  80
Asn Leu Lys His Gln Pro Gly Gly Gly Lys Val Gln Ile Ile Asn Lys
                85                  90                  95
Lys Leu Asp Leu Ser Asn Val Gln Ser Lys Cys Gly Ser Lys Asp Asn
            100                 105                 110
Ile Lys His Val Pro Gly Gly Gly Ser Val Gln Ile Val Tyr Lys Pro
        115                 120                 125
Val Asp Leu Ser Lys Val Thr Ser Lys Cys Gly Ser Leu Gly Asn Ile
    130                 135                 140
His His Lys Pro Gly Gly Gly Gln Val Glu Val Lys Ser Glu Lys Leu
145                 150                 155                 160
Asp Phe Lys Asp Arg Val Gln Ser Lys Ile Gly Ser Leu Asp Asn Ile
                165                 170                 175
Thr His Val Pro Gly Gly Gly Asn Lys Lys Ile Glu Thr His Lys Leu
            180                 185                 190
Thr Phe Arg Glu Asn Ala Lys Ala Lys Thr Asp His Gly Ala Glu Ile
        195                 200                 205
Val Tyr Lys Ser Pro Val Val Ser Gly Asp Thr Ser Pro Arg His Leu
    210                 215                 220
Ser Asn Val Ser Ser Thr Gly Ser Ile Asp Met Val Asp Ser Pro Gln
225                 230                 235                 240
Leu Ala Thr Leu Ala Asp Glu Val Ser Ala Ser Leu Ala Lys Gln Gly
                245                 250                 255
Leu

<210>   2
<211>   64
<212>   PRT
<213>   ADARB1[678-741]
<400>   2
Arg Val His Gly Lys Val Pro Ser His Leu Leu Arg Ser Lys Ile Thr
1               5                   10                  15
Lys Pro Asn Val Tyr His Glu Ser Lys Leu Ala Ala Lys Glu Tyr Gln
            20                  25                  30
Ala Ala Lys Ala Arg Leu Phe Thr Ala Phe Ile Lys Ala Gly Leu Gly
        35                  40                  45
Ala Trp Val Glu Lys Pro Thr Glu Gln Asp Gln Phe Ser Leu Thr Pro
    50                  55                  60

<210>   3
<211>   573
<212>   PRT
<213>   HSP60[1-573]
<400>   3

<210>  4
<211>  163
<212>  PRT
<213>  P21[2-164]
<400>  4
Ser Glu Pro Ala Gly Asp Val Arg Gln Asn Pro Cys Gly Ser Lys Ala
1               5                   10                  15
Cys Arg Arg Leu Phe Gly Pro Val Asp Ser Glu Gln Leu Ser Arg Asp
            20                  25                  30
Cys Asp Ala Leu Met Ala Gly Cys Ile Gln Glu Ala Arg Glu Arg Trp
        35                  40                  45
Asn Phe Asp Phe Val Thr Glu Thr Pro Leu Glu Gly Asp Phe Ala Trp
    50                  55                  60
Glu Arg Val Arg Gly Leu Gly Leu Pro Lys Leu Tyr Leu Pro Thr Gly
65                  70                  75                  80
Pro Arg Arg Gly Arg Asp Glu Leu Gly Gly Gly Arg Arg Pro Gly Thr
                85                  90                  95
Ser Pro Ala Leu Leu Gln Gly Thr Ala Glu Glu Asp His Val Asp Leu
            100                 105                 110
Ser Leu Ser Cys Thr Leu Val Pro Arg Ser Gly Glu Gln Ala Glu Gly
        115                 120                 125
Ser Pro Gly Gly Pro Gly Asp Ser Gln Gly Arg Lys Arg Arg Gln Thr
    130                 135                 140
Ser Met Thr Asp Phe Tyr His Ser Lys Arg Arg Leu Ile Phe Ser Lys
145                 150                 155                 160
Arg Lys Pro


<210>  5
<211>  9
<212>  PRT
<213>  DAG[1-9]
<400>  5
Cys Asp Ala Gly Arg Lys Gln Lys Cys
1               5

<210>  6
<211>  140
<212>  PRT
<213>  DNAJC8[114-253]
<400>  6
Glu Gln Lys Lys Arg Ala Leu Asp Val Ile Gln Ala Gly Lys Glu Tyr
1               5                   10                  15
Val Glu His Thr Val Lys Glu Arg Lys Lys Gln Leu Lys Lys Glu Gly
            20                  25                  30
Lys Pro Thr Ile Val Glu Glu Asp Asp Pro Glu Leu Phe Lys Gln Ala
        35                  40                  45
Val Tyr Lys Gln Thr Met Lys Leu Phe Ala Glu Leu Glu Ile Lys Arg
    50                  55                  60
Lys Glu Arg Glu Ala Lys Glu Met His Glu Arg Lys Arg Gln Arg Glu
65                  70                  75                  80
Glu Glu Ile Glu Ala Gln Glu Lys Ala Lys Arg Glu Arg Glu Trp Gln
                85                  90                  95

Lys Asn Phe Glu Glu Ser Arg Asp Gly Arg Val Asp Ser Trp Arg Asn
            100                 105                 110
Phe Gln Ala Asn Thr Lys Gly Lys Lys Glu Lys Lys Asn Arg Thr Phe
        115                 120                 125
Leu Arg Pro Pro Lys Val Lys Met Glu Gln Arg Glu
    130                 135                 140

<210>  7
<211>  32
<212>  PRT
<213>  RAGE[23-54]
<400>  7
Ala Gln Asn Ile Thr Ala Arg Ile Gly Glu Pro Leu Val Leu Lys Cys
1               5                   10                  15
Lys Gly Ala Pro Lys Lys Pro Pro Gln Arg Leu Glu Trp Lys Leu Asn
            20                  25                  30

<210>  8
<211>  84
<212>  PRT
<213>  ASXL1[1-84]
<400>  8
Met Lys Asp Lys Gln Lys Lys Lys Lys Glu Arg Thr Trp Ala Glu Ala
1               5                   10                  15
Ala Arg Leu Val Leu Glu Asn Tyr Ser Asp Ala Pro Met Thr Pro Lys
            20                  25                  30
Gln Ile Leu Gln Val Ile Glu Ala Glu Gly Leu Lys Glu Met Ser Gly
        35                  40                  45
Thr Ser Pro Leu Ala Cys Leu Asn Ala Met Leu His Ser Asn Ser Arg
    50                  55                  60
Gly Gly Glu Gly Leu Phe Tyr Lys Leu Pro Gly Arg Ile Ser Leu Phe
65                  70                  75                  80
Thr Leu Lys Arg

<210>  9
<211>  160
<212>  PRT
<213>  JMJD2D[1-160]
<400>  9
Met Glu Thr Met Lys Ser Lys Ala Asn Cys Ala Gln Asn Pro Asn Cys
1               5                   10                  15
Asn Ile Met Ile Phe His Pro Thr Lys Glu Glu Phe Asn Asp Phe Asp
            20                  25                  30
Lys Tyr Ile Ala Tyr Met Glu Ser Gln Gly Ala His Arg Ala Gly Leu
        35                  40                  45
Ala Lys Ile Ile Pro Pro Lys Glu Trp Lys Ala Arg Glu Thr Tyr Asp
    50                  55                  60
Asn Ile Ser Glu Ile Leu Ile Ala Thr Pro Leu Gln Gln Val Ala Ser
65                  70                  75                  80
Gly Arg Ala Gly Val Phe Thr Gln Tyr His Lys Lys Lys Lys Ala Met
                85                  90                  95
Thr Val Gly Glu Tyr Arg His Leu Ala Asn Ser Lys Lys Tyr Gln Thr
            100                 105                 110
Pro Pro His Gln Asn Phe Glu Asp Leu Glu Arg Lys Tyr Trp Lys Asn
        115                 120                 125
Arg Ile Tyr Asn Ser Pro Ile Tyr Gly Ala Asp Ile Ser Gly Ser Leu
    130                 135                 140
Phe Asp Glu Asn Thr Lys Gln Trp Asn Leu Gly His Leu Gly Thr Ile
145                 150                 155                 160

## Description

### CROSS-REFERENCE TO RELATED APPLICATIONS

This application is a continuation of International Pat. Appl. No. PCT/CN2020/085224, filed April 17, 2020, pending, which claims the benefit of Chinese Patent Application No. 202010039229.1, filed on January 15, 2020, incorporated herein by reference in its entirety.

### FIELD OF THE INVENTION

The present disclosure belongs to the field of biological detection, in particular to a protein antigen combination for detecting Alzheimer's disease autoantibodies in human serum sample and an application thereof.

### BACKGROUND

Alzheimer's disease (AD) is a progressive neurodegenerative disease characterized by memory and cognitive dysfunction. It frequently occurs in the elderly, with a slow and irreversible course. According to the development of cognitive dysfunction, Alzheimer's disease can be divided into early, middle and late stages. In the early stage, the patients have no obvious symptoms, most of them only show forgetfulness and anxiety, so it is difficult to detect or diagnose. In the middle stage, there will be chaotic symptoms, the patients' character and habits will change, and memory will also be confused. In the later stage, the patients completely show dementia, have no ability to take care of themselves, and eventually die of a variety of secondary diseases.

The current diagnostic methods of Alzheimer's disease include: 1. Early and significant episodic memory dysfunction; 2. Middle temporal gyrus atrophy; 3. Abnormal cerebrospinal fluid biomarkers, including decreased concentration of amyloid beta 1-42 (Aβ 1-42), increased concentration of total Tau protein, increased concentration of phosphorylated Tau protein, or a combination of the three; 4. Specificity of PET functional neuroimaging showed that glucose metabolic rate in bilateral temporal and parietal lobes decreased; 5. AD related autosomal dominant mutation was found in lineal relatives. However, because the early symptoms are not obvious, and it is difficult to distinguish the middle stage from other diseases, there are still significant problems of missed diagnosis and misdiagnosis in the diagnosis of Alzheimer's disease. Therefore, an effective tool for the diagnosis or staging of AD is urgently needed.

An autoantibody is an antibody produced by the individual's immune system against the individual's autoprotein (i.e., own protein) antigen. Normally, the immune system produces antibodies in response to exogenous proteins or substances in the body, but sometimes it also recognizes one or more endogenous components of the body, resulting in the production of autoantibody. There has been a lot of evidence that there are a variety of autoantibodies in the serum involved in neurological diseases and syndromes. As far as Alzheimer's disease is concerned, many literatures have reported that patients have high titers of autoantibodies against non-brain and brain-related targets, including those binding to neurons.

The detection of antibodies in serum is a very mature technology, which makes it possible to accurately detect the autoantibodies of Alzheimer's disease, and then use the autoantibodies of Alzheimer's disease in serum as the detection target to achieve the purpose of diagnosis. For example, Chinese patent application Publication No. CN103154736A disclosed a series of proteins, which suggested that these proteins could potentially be used as antigens binding to autoantibodies of Alzheimer's disease, so they could be used as diagnostic indicators of Alzheimer's disease.

However, the protein antigens disclosed in CN103154736Aare extremely numerous, so it is still necessary to select the proteins that can be used in combination as a protein antigen combination to detect the possible autoantibodies of Alzheimer's disease in the serum of subjects with high specificity and sensitivity, so as to provide a practical means for the diagnosis of Alzheimer's disease. Moreover, even for a single protein antigen, the binding of the full-length protein to the corresponding autoantibodies of Alzheimer's disease may be very weak, resulting in poor detection, detection failure, or even misdiagnosis. Therefore, whether used alone or in combination, it is very important to screen out specific protein antigen combination, or specific region or fragment in the protein to effectively bind and detect the corresponding autoantibodies.

### SUMMARY OF THE INVENTION

An object of the invention is to provide a protein antigen combination for detecting Alzheimer's disease autoantibodies in human serum sample and its application.

In order to realize the above object of the invention, embodiments of the invention include an antigen combination that includes at least two protein fragments selected from the following proteins: MAPT, ADARB 1, P21, DNAJC8, RAGE, ASXL1 and JMJD2D.

In some embodiments, the protein fragment sequence is selected from one or more of the following sequences:
The MAPT protein fragment shown in SEQ ID No.1;
The Adarb1 protein fragment shown in SEQ ID No.2;
The P21 protein fragment shown in SEQ ID No.4;
The DNAJC8 protein fragment shown in SEQ ID No.6;
The RAGE protein fragment shown in SEQ ID No.7;
The ASXL1 protein fragment shown in SEQ ID No.8; and
The JMJD2D protein fragment shown in SEQ ID No.9.

Furthermore, the protein antigen combination may comprise any one of the following proteins: HSP60 and DAG. The protein sequences may be as follows: the HSP60 protein sequence shown in SEQ ID No.3; and the DAG protein sequence shown in SEQ ID No.5.

In some embodiments, the protein antigen combination includes the MAPT protein fragment, the ADARB 1 protein fragment and the HSP60 protein.

In other embodiments, the protein antigen combination includes the DNAJC8 protein fragment, the RAGE protein fragment and the ASXL1 protein fragment.

In other embodiments, the protein antigen combination includes the JMJD2D protein fragment, the P21 protein fragment and the DAG protein.

In yet other embodiments, the protein antigen combination includes (i) the MAPT protein fragment, the HSP60 protein and the DNAJC8 protein fragment; (ii) the ADARB1 protein fragment, the RAGE protein fragment and the ASXL1 protein fragment; or (iii) the HSP60 protein, the RAGE protein fragment and the P21 protein fragment.

Accordingly, the protein antigen combination can be applied in detecting Alzheimer's disease.

Accordingly, a kit for detecting Alzheimer's disease can be prepared by using the protein antigen combination.

Accordingly, the sample used in the kit may be whole blood or serum.

Preferably, an antigen for detecting Alzheimer's disease is a MAPT protein fragment, and its sequence may be as shown in SEQ ID No.1.

Another antigen for detecting Alzheimer's disease is an ADARB 1 protein fragment, and its sequence may be shown in SEQ ID No.2.

Another antigen for detecting Alzheimer's disease is a P21 protein fragment, and its sequence may be shown in SEQ ID No.4.

Another antigen for detecting Alzheimer's disease is a DNAJC8 protein fragment, and its sequence may be shown in SEQ ID No.6.

Another antigen for detecting Alzheimer's disease is a RAGE protein fragment, and its sequence may be shown in SEQ ID No.7.

Another antigen for detecting Alzheimer's disease is an ASXL1 protein fragment, and its sequence may be shown in SEQ ID No.8.

Yet another antigen for detecting Alzheimer's disease is a JMJD2D protein fragment, and its sequence may be shown in SEQ ID No.9.

The invention has the following beneficial effects: The invention provides a protein antigen composition for detecting the autoantibodies of Alzheimer's disease, wherein the protein antigen or its fragment can be prepared by artificial synthesis. For example, the synthetic protein antigen or the coding DNA of its fragment is used as the template, the primers are designed, and the gene fragment of the protein antigen or its fragment is cloned into an expression plasmid by PCR, enzyme digestion, ligation and other molecular cloning means. At the same time, HIS and c-myc tags can be selectively added to the N-terminal of protein antigen or its fragment.

The protein antigen combination provided by the invention has the following uses:
(1) Testing the presence or presence level of autoantibodies against the above protein antigen combination in biological samples from the subject, so as to determine whether the subject has Alzheimer's disease; predicting whether the subject has a risk of Alzheimer's disease; evaluating the progress of Alzheimer's disease; or determining whether the subject has the risk of recurrence of Alzheimer's disease. The biological samples can be serum, plasma, whole blood, saliva, oral mucosa swab, urine, lymph, cerebrospinal fluid, etc. According to the specific situation, the biological samples can be pretreated by extraction, dilution, enrichment and other means. The method of use is various, simple and easy to operate.
   When the protein antigen combination provided by the invention is used for the above test, the presence or presence level of the autoantibody is tested by binding or interacting the protein or fragment in the protein antigen combination with the corresponding autoantibody that may exist.
(2) The protein antigen combination provided by the invention can also be used for preparing Alzheimer's disease autoantibody detection reagents or Alzheimer's disease diagnosis reagents. It should be understood that the protein antigen combination can also be used to prepare an Alzheimer's disease autoantibody detection kit. The kit can refer to the method and reagent preparation when using the protein antigen combination for Alzheimer's disease autoantibody detection in embodiments of the invention, and can also be adjusted according to the needs.

In conclusion, the invention provides a protein antigen combination, which can be used for the detection or diagnosis of Alzheimer's disease, especially for early detection or diagnosis; it can also be used for the disease risk prediction of Alzheimer's disease; and it can be further prepared as a related reagent or kit according to the needs.

### DETAILED DESCRIPTION

The present application is further explained in combination with specific embodiments. Unless otherwise specified, the experimental methods used in the following examples are conventional methods; the materials and reagents used can be obtained from commercial channels; the data obtained are the average values obtained after at least three repetitions, and the data obtained from each repetition are valid data.

### Example 1: construction, expression and purification of recombinant vector of protein antigen

1. Protein antigen selection. 115 antigen proteins highly related to Alzheimer's disease were selected for construction, expression and purification. For brevity, only 50 representative ones of them are represented in this paper, and their respective database IDs are shown in Table 1.

**Table 1:Database ID of proteins to be tested**

| Protein name | Database ID | Protein name | Database ID |
|---|---|---|---|
| RABPT5 | NM_004703.6 | C20orf96 | BC051762.1 |
| RAGE | NM_001136.5 | CCL12 | NM_011331.2 |
| MAPT | NM_016835.4 | ARPP-19 | NM_006628.4 |
| MBP | NM_001025101.2 | BMF | BC069328.1 |
| MOG | NM_002433.4 | CA13 | BC052602.1 |
| PTCD2 | NM_024754.5 | CENTD2 | BC056401.1 |
| POMC | NM_000939.4 | CSAG3A | BC007228.1 |
| DNAJC8 | NM_014280 | CSNK2A2 | NM_001896.2 |
| MRPL34 | NM_023937.3 | DDX54 | BC001132.1 |
| CENTA2 | NM_018404 | FCGR3A | BC036723.1 |
| HSP60 | NM_002156.5 | FGF16 | NM_003 868.1 |
| ADARB 1 | NM_001112.4 | HSPC111 | BC040106.1 |
| LENG1 | NM_024316.2 | IER3 | NM_003897.2 |
| ASXL1 | NM_015338.5 | IMPACT | NM_018439.1 |
| RPL23A | NM_000984.6 | PIN4 | NM_006223.1 |
| EDRK | NP_001171558.1 | ROBO3 | BC008623.1 |
| P21 | NM_000389 | SERPINE2 | BC042628.1 |
| GDF11 | NM_005811 | SPT16 | BC021561.1 |
| CCL2 | NM_002982.4 | STK25 | NM_006374.2 |
| JMJD2D | NM_00018039.3 | TARBP2 | NM_004178.3 |
| GFAP | NM_002055.5 | TBC1D21 | NM_153356.1 |
| DAG | NM_004393.5 | TIPIN | BC000870.1 |
| CTGF | NM_001901.2 | TTLL7 | BC048970.1 |
| SH3GL2 | NM_003026.3 | VCY | BC056508.1 |
| RABEP2 | BC058900.1 | POLB | NM_002690.1 |

2.Construction and expression of antigen recombinant vector. The target protein antigens were screened from the 50 proteins in Table 1. Using a human cDNA library (purchased from Invitrogen company) or whole gene synthetic DNA as template, primers were designed respectively, and the full-length gene of the proteins was cloned into the pET28 plasmid by PCR, enzyme digestion, ligation and other molecular cloning methods. At the same time, HIS and c-myc tags were added to the N-terminal of the proteins to form fusion proteins. The recombinant expression vector was identified by DNA sequencing and confirmed to contain the correct protein gene fragment. It should be noted that the added tag(s)is only convenient for identification and extraction of protein, and does not have a decisive impact on the function of protein as antigen. When using, it is not necessary to add a label or add other labels as needed.
The recombinant plasmid containing the protein gene fragment was transformed into competent cells of E. coli BL21 (DE3), and the clones were selected and inoculated into LB medium, and then shaking-cultured at 37°Cto grow the bacteria. When the E. coli BL21 (DE3) density reached OD₆₀₀ about 0.8, the temperature was reduced to 16°C, and 0.1mM isopropylthio - β - d-galactoside (IPTG) was added to each LB medium to induce the expression overnight.
3.Purification of protein antigen. The expression-induced bacteria were collected by centrifugation and rinsed twice with PBS. The pyrolysate (5-10mLpyrolysate per g bacteria) was used to suspend and disperse the bacteria. The bacteria cell was broken by ice bath and ultrasonic wave (ultrasonic power 200W, broken for 5S, rest for 5S). After crushing, centrifugation at 13000rpm,10°Cwas conducted for, and the supernatant was purified by Ni column affinity chromatography and molecular sieve chromatography. SDS-PAGE electrophoresis was used to confirm the molecular weight and purity of the protein. Bradford method was used to determine the concentration, and then the protein was stored at -80°C for standby. The purified protein was obtained.

### Example 2: screening candidate antigens from proteins to be tested

1.The solutions and reagents used in this example are as follows:
(1) The coating buffer was PBS buffer, pH=7.4. The preparation method is as follows: 3.58g Na₂HPO₄·12H₂O, 0.23g KH₂PO₄·2H₂O, 0.2g KCl and 8.0g NaCl are accurately weighed and dissolved in a relatively small amount of water, then diluted with water to 1L.
(2) Blocking solution / sample diluent / antibody diluent: dissolve 10g BSA (bovine serum albumin) in the coating buffer, and dilute with water to 1L.
(3) Detergent: It should be prepared when using. Add 0.5% Tween20(V/V) to the coating buffer before use, pH=7.4.
(4) TMB chromogenic agent, purchased from KPL company.
(5) Termination solution: 1M hydrochloric acid.

2.Coatingthe protein onto a solid phase:Dilute the purified protein to be tested in example 1 to 5µg/mL with the coating buffer, add it to 96-well plate, 50µL for each well, and coat it at 4°C overnight. The next day, pour out the solution, dry it, and wash it three times with detergent, 200µL per well each time. Then 200µL blocking solution was added to each well, and incubated at room temperature for 1h, then the blocking solution was poured out and dried, and then washed with the detergent for three times, 200µL per well each time, and dried again to obtain the solid coated antigen in 96 well plate.
3.Adding the sample to be tested: The human serum to be tested was diluted 100 times with sample diluent and then added to the 96-well plate containing the protein to be tested, and 50µL of the diluted sample to be tested is added to each well. Then, the 96-well plate was placed on the microplate shaker and incubated at room temperature for 1h; then, the plate was dried, washed with the detergent three times, 200µL per well each time, and then dried again.
4. Adding the enzyme-labeled second antibody: 1.0mg/mL horseradish peroxidase-labeled recombinant Goat anti-human immunoglobulin G antibody (purchased from Jackson immune research Inc.) was diluted 20000 times with the antibody diluent, and then added to the 96-well plate treated in step 3, 50µL for each well. Then, the 96-well plate was placed on the microplate shaker, incubated at room temperature for 0.5h, then dried, washed three times with the detergent, 200µL per well each time, and dried again.
5. Color reaction and optical density reading: In the 96-well plate treated in step 4, 50µLTMB chromogenic agent was added to each well, and the shaking time was 15s. The reaction was kept away from light for 15min at room temperature, and then 50µL termination solution was added. Then the absorbance value at 450nm was read by a microplate reader to obtain the detection signal(S) of each sample.
6. Sensitivity and specificity analysis: 180 positive samples (serum of patients diagnosed with Alzheimer's disease) and 180 negative samples (serum of healthy subjects) were taken respectively. The detection signal(S) of each sample was determined according to the above method (absorption value of 450nm wavelength). Negative samples were taken as negative reference samples. The mean (M, average value) and standard deviation (SD) of the detection signals (S) of all negative reference samples were calculated, and M + 3SD was taken as the cut-off value. The samples with a detection signal(S) ≥ cut off value (S ≥ M + 3SD) were regarded as positive; the samples with a detection signal (S) < cut off value (S < M + 3SD) were regarded as negative.
The specificity and sensitivity were calculated based on the positive and negative results of the samples. Among them, specificity refers to the proportion of healthy subjects whose samples are correctly determined as negative, that is, the number of negative samples which are correctly determined as negative divided by the total number of negative samples. Sensitivity refers to the proportion of samples of patients with Alzheimer's disease that are correctly determined to be positive, that is, the number of positive samples divided by the total number of positive samples. The sensitivity and specificity of using each protein to be tested as antigen for sample detection are calculated, and the results are shown in Table 2.

**Table 2:Sensitivity and specificity of proteins to be tested as antigens**

| protein | susceptibility | Specificity | protein | susceptibility | Specificity |
|---|---|---|---|---|---|
| RABPT5 | 20.56% | 83.33% | C20orf96 | 7.78% | 79.44% |
| RAGE | 28.33% | 84.44% | CCL12 | 11.11% | 93.33% |
| MAPT | 36.67% | 88.89% | ARPP-19 | 18.89% | 76.11% |
| MBP | 27.78% | 89.44% | BMF | 16.67% | 82.22% |
| MOG | 20.56% | 80.56% | CA13 | 8.89% | 81.67% |
| PTCD2 | 24.44% | 84.44% | CENTD2 | 4.44% | 99.44% |
| POMC | 21.11% | 85.56% | CSAG3A | 11.67% | 72.22% |
| DNAJC8 | 31.11% | 86.67% | CSNK2A2 | 17.22% | 79.44% |
| MRPL34 | 26.67% | 87.22% | DDX54 | 0.56% | 76.11% |
| CENTA2 | 27.22% | 82.78% | FCGR3A | 0.56% | 95% |
| HSP60 | 35.56% | 88.33% | FGF16 | 4.44% | 84.44% |
| ADARB 1 | 36.67% | 87.78% | HSPC111 | 18.89% | 72.22% |
| LENG1 | 24.44% | 87.22% | IER3 | 19.44% | 90% |
| ASXL1 | 27.78% | 86.11% | IMPACT | 16.11% | 74.44% |
| RPL23A | 22.22% | 83.33% | PIN4 | 5.56% | 95% |
| EDRK | 26.67% | 85.56% | ROBO3 | 8.33% | 85% |
| P21 | 36.11% | 84.44% | SERPINE2 | 15% | 91.11% |
| GDF11 | 25% | 84.44% | SPT16 | 10% | 97.22% |
| CCL2 | 25.56% | 86.67% | STK25 | 19.44% | 80% |
| JMJD2D | 27.78% | 86.11% | TARBP2 | 11.11% | 73.89% |
| GFAP | 23.89% | 85% | TBC1D21 | 10% | 77.78% |
| DAG | 30.56% | 85.56% | TIPIN | 2.78% | 85.56% |
| CTGF | 12.22% | 86.67% | TTLL7 | 19.44% | 84.44% |
| SH3GL2 | 18.33% | 78.89% | VCY | 10% | 97.78% |
| RABEP2 | 23.89% | 68.89% | POLB | 14.44% | 80.56% |

7.The proteins with sensitivity ≥ 20% and specificity ≥ 75% were selected as candidate antigens. The screening results are shown in Table 3.

**Table 3:Display of candidate antigens selected from the proteins to be tested**

| Candidate antigen | susceptibility | Specificity |
|---|---|---|
| MAPT | 36.67% | 88.89% |
| ADARB 1 | 36.67% | 87.78% |
| HSP60 | 35.56% | 88.33% |
| P21 | 36.11% | 84.44% |
| DAG | 30.56% | 85.56% |
| DNAJC8 | 31.11% | 86.67% |
| RAGE | 28.33% | 84.44% |
| ASXL1 | 27.78% | 86.11% |
| JMJD2D | 27.78% | 86.11% |
| EDRK | 26.67% | 85.56% |
| MBP | 27.78% | 89.44% |
| MRPL34 | 26.67% | 87.22% |
| CENTA2 | 27.22% | 82.78% |
| PTCD2 | 24.44% | 84.44% |
| LENG1 | 24.44% | 87.22% |
| GDF11 | 25.00% | 84.44% |
| CCL2 | 25.56% | 86.67% |
| GFAP | 23.89% | 85.00% |
| RPL23A | 22.22% | 83.33% |
| POMC | 21.11% | 85.56% |
| RABPT5 | 20.56% | 83.33% |
| MOG | 20.56% | 80.56% |

### Example 3: active sites screening of candidate antigens

1.The amino acid sequences and structures of the candidate antigens obtained in example 2 were analyzed. After a large number of preliminary tests, different sequence fragments and epitopes were selected from each candidate antigen. The selected sequence fragments or full length is shown in Table 4.

**Table 4:Sequence fragments intercepted from each candidate antigen**

| Candidate antigen | Starting and ending positions of amino acids | Candidate antigen | Starting and ending positions of amino acids |
|---|---|---|---|
| MAPT | 2-288 | DNACJ8 | 2-253 |
| | 502-758 | | 114-253 |
| | 526-691 | | 2-140 |
| ADARB 1 | 1-299 | RAGE | 1-404 |
| | 299-741 | | 23-54 |
| | 678-741 | | 338-404 |
| HSP60 | 1-355 | ASXL1 | 1-84 |
| | 302-573 | | 100-209 |
| | 1-573 | | 641-684 |
| P21 | 42-164 | JMJD2D | 1-523 |
| | 2-164 | | 223-523 |
| | 55-160 | | 1-160 |
| EDRK | 1-35 | GDF11 | 25-298 |
| | 20-59 | | 42-407 |
| | 1-62 | | 299-407 |
| MBP | 120-304 | CCL2 | 45-120 |
| | 1-304 | | 1-120 |
| | 1-135 | | 25-100 |
| MRPL34 | 1-92 | GFAP | 120-423 |
| | 1-46 | | 100-175 |
| | 47-92 | | 251-436 |
| CENTA2 | 1-220 | RPL23A | 1-163 |
| | 105-320 | | 81-163 |
| | 1-381 | | 9-163 |
| PTCD2 | 151-268 | POMC | 61-301 |
| | 38-120 | | 1-301 |
| | 195-320 | | 113-301 |
| LENG1 | 1-264 | RABPT5 | 1-433 |
| | 1-120 | | 101-399 |
| | 121-260 | | 187-433 |
| MOG | 1-252 | DAG | 1-9 |
| | 30-183 | | |
| | 30-252 | | |

2.According to the method of example 1, the expression vector of each protein fragment in Table 4 was constructed, expressed and purified to obtain the constructed protein fragment, and then the sensitivity and specificity of each constructed protein fragment as antigen were detected according to the method of example 2.As in example 1, although some labels are added to each protein fragment, the purpose of adding labels is to facilitate extraction and recognition, and does not have a material impact on the function of protein fragment as antigen. The purpose of the invention can be realized by using the protein before and after adding the label. In actual use, a person having ordinary skill in the art can select whether to add the label and the type of the label according to the need. Therefore, the full text of the invention only provides the protein sequence before labeling. The test results are shown in Table 5.

**Table 5: Comparison table of sensitivity and specificity of optimized candidate antigen fragments**

| Candidate antigen | Starting and ending positions of amino acids | susceptibility | Specificity |
|---|---|---|---|
| MAPT | 2-288 | 11.11% | 83.33% |
| | 502-758 | 40.56% | 90.00% |
| | 526-691 | 19.44% | 80.00% |
| ADARB 1 | 1-299 | 24.44% | 72.22% |
| | 299-741 | 42.78% | 83.89% |
| | 678-741 | 39.44% | 93.33% |
| HSP60 | 1-355 | 15.56% | 84.44% |
| | 302-573 | 20.00% | 88.89% |
| | 1-573 | 38.33% | 95.00% |
| P21 | 42-164 | 27.78% | 88.89% |
| | 2-164 | 37.22% | 90.00% |
| | 55-160 | 30.56% | 89.44% |
| DAG | 1-9 | 35.56% | 90.00% |
| DNACJ8 | 2-253 | 32.22% | 81.67% |
| | 114-253 | 35.00% | 88.89% |
| | 2-140 | 50.00% | 55.56% |
| RAGE | 1-404 | 20.56% | 81.67% |
| | 23-54 | 33.89% | 88.33% |
| | 338-404 | 24.44% | 82.78% |
| ASXL1 | 1-84 | 33.33% | 90.00% |
| | 100-209 | 31.67% | 79.44% |
| | 641-684 | 32.78% | 82.22% |
| JMJD2D | 1-523 | 20.56% | 73.89% |
| | 223-523 | 23.89% | 77.78% |
| | 1-160 | 33.89% | 89.44% |
| EDRK | 1-35 | 21.11% | 77.78% |
| | 20-59 | 23.33% | 83.89% |
| | 1-64 | 32.22% | 90.00% |
| MBP | 120-304 | 32.22% | 87.22% |
| | 1-304 | 27.78% | 84.44% |
| | 1-135 | 26.11% | 85.56% |
| MRPL34 | 1-92 | 19.44% | 90.56% |
| | 1-46 | 23.89% | 81.67% |
| | 47-92 | 30.56% | 87.22% |
| CENTA2 | 1-220 | 22.78% | 80.00% |
| | 105-320 | 25.56% | 75.56% |
| | 1-381 | 30.00% | 88.89% |
| PTCD2 | 151-268 | 31.11% | 77.22% |
| | 38-120 | 28.89% | 91.11% |
| | 195-320 | 21.67% | 86.67% |
| LENG1 | 1-264 | 27.78% | 92.22% |
| | 1-120 | 25.00% | 85.00% |
| | 121-260 | 26.11% | 85.56% |
| GDF11 | 25-298 | 25.56% | 78.33% |
| | 42-407 | 27.78% | 92.78% |
| | 299-407 | 23.89% | 86.11% |
| CCL2 | 45-120 | 26.67% | 87.22% |
| | 1-120 | 10.56% | 91.11% |
| | 25-100 | 21.11% | 81.67% |
| GFAP | 120-423 | 26.11% | 89.44% |
| | 100-175 | 19.44% | 84.44% |
| | 251-436 | 26.11% | 75.00% |
| RPL23A | 1-163 | 8.89% | 92.22% |
| | 81-163 | 17.22% | 86.67% |
| | 9-163 | 25.56% | 88.33% |
| POMC | 61-301 | 23.89% | 87.22% |
| | 1-301 | 15.00% | 93.33% |
| | 113-301 | 20.56% | 82.78% |
| RABPT5 | 1-433 | 18.89% | 79.44% |
| | 101-399 | 16.67% | 84.44% |
| | 187-433 | 22.22% | 89.44% |
| MOG | 1-252 | 8.33% | 93.33% |
| | 30-183 | 20.00% | 83.33% |
| | 30-252 | 13.89% | 80.56% |

3. According to the results in Table 5, for some proteins, compared with the whole protein, the protein fragments obtained by intercepting specific amino acid sequence regions and corresponding optimization have better detection effect; for some antigens, the detection effect of whole protein is better than that of its fragments. This may be due to the inhibition of some protein fragments in some whole proteins during the binding or recognition of Alzheimer's disease-related sites.

According to the results in Table 5, nine full-length proteins or their fragments were further screened. The sensitivity of the selected full-length proteins or their fragments was more than 33%, and the specificity was more than 88%. They were used as candidate antigens to distinguish Alzheimer's disease patients and healthy subjects. The screening results are shown in Table 6.

**Table 6: Comparison of sensitivity and specificity of each candidate antigen**

| Candidate antigen | susceptibility | Specificity | Fragment or full length | Serial number |
|---|---|---|---|---|
| MAPT[502-758] | 40.56% | 90.00% | fragment | SEQ ID NO.1 |
| ADARB1[678-741] | 39.44% | 93.33% | fragment | SEQ ID NO.2 |
| HSP60[1-573] | 38.33% | 95.00% | overall length | SEQ ID NO.3 |
| P21[2-164] | 37.22% | 90.00% | fragment | SEQ ID NO.4 |
| DAG[1-9] | 35.56% | 90.00% | overall length | SEQ ID NO.5 |
| DNAJC8[114-253] | 35.00% | 88.89% | fragment | SEQ ID NO.6 |
| RAGE[23-54] | 33.89% | 88.33% | fragment | SEQ ID NO.7 |
| ASXL1[1-84] | 33.33% | 90.00% | fragment | SEQ ID NO.8 |
| JMJD2D[1-160] | 33.89% | 89.44% | fragment | SEQ ID NO.9 |

### Example 4: display of detection effect of candidate antigens

1.According to the screening results in Table 6, different antigen combinations are selected from the candidate protein antigens. Based on the whole proteins corresponding to the candidate protein fragments of candidate groups 1-8, the corresponding whole protein combinations are set as control groups 1-8; according to the combinations 5 and 6 in Table 5 of patent CN109738653A, the control groups 9 and 10 are set respectively. The details are shown in Table 7.It should be noted that the inventor did not only carry out the combination test in Table 7; the inventor obtained the protein antigen combinations shown in Table 7 after a large number of preliminary tests, and only selected some combinations with better effect due to space limitation.

**Table 7: Comparison of antigen combinations**

| group | combination |
|---|---|
| Candidate group 1 | MAPT[502-758],ADARB1[678-741],HSP60[1-573] |
| Candidate group 2 | DNAJC8[114-253],RAGE[23-54],ASXL1[1-84] |
| Candidate group 3 | JMJD2D[1-160],P21[2-164],DAG[1-9] |
| Candidate group 4 | MAPT[502-758],DNAJC8[114-253],JMJD2D[1-160] |
| Candidate group 5 | MAPT[502-758],HSP60[1-573],DNAJC8[114-253] |
| Candidate group 6 | ADARB1[678-741],RAGE[23-54],ASXL1[1-84] |
| Candidate group 7 | HSP60[1-573],RAGE[23-54],P21[2-164] |
| Candidate group 8 | ADARB1[678-741],HSP60[1-573],ASXL1[1-84] |
| Candidate group 9 | MAPT[502-758],RAGE[23-54],JMJD2D[1-160] |
| Candidate group 10 | ADARB1[678-741],ASXL1[1-84],DAG[1-9] |
| Control group 1 | MAPT,ADARB1,HSP60 |
| Control group 2 | DNAJC8,RAGE,ASXL1 |
| Control group 3 | JMJD2D,P21,DAG |
| Control group 4 | MAPT,DNAJC8,JMJD2D |
| Control group 5 | MAPT,HSP60,DNAJC8 |
| Control group 6 | ADARB 1,RAGE,ASXL1 |
| Control group 7 | HSP60,RAGE,P21 |
| Control group 8 | ADARB 1,HSP60,ASXL1 |
| Control group 9 | RAGE,DNAJC8,HSP60,MRPL34,ADARB 1,CCL2,MAPT,ASXL1, GDF11 |
| Control group 10 | RAGE,DNAJC8,HSP60,MRPL34,ADARB 1,CCL2,MAPT,MBP,G DF11 |

2.According to Mini-Mental State Examination (MMSE),the patients with Alzheimer's disease and healthy subjects were scored. The subjects with scores of 27-30 were normal, the subjects with scores of 21-26 were mild (early), the subjects with scores of 10-20 were moderate (middle), and the subjects with scores of 0-9 were severe (late).

According to the MMSE score, 180 patients with Alzheimer's disease in the early stage, 90 patients in the middle stage and 90 patients in the late stage were selected as the test samples; 180 healthy subjects with normal MMSE score were selected as the samples of healthy subjects. According to the detection method of example 2, the sensitivity and specificity of the above samples were detected by using the protein antigen combinations of each group in Table 7 (it should be noted that the proteins used in the control group were whole proteins).The sensitivity and specificity in this embodiment are specifically overall specificity, overall sensitivity, early sensitivity, mid-term sensitivity and late sensitivity.

Among them, the definition method of positive and negative for early sensitivity, mid-term sensitivity and late sensitivity refers to example 2.

Early sensitivity refers to the proportion of 180 samples of patients with early Alzheimer's disease correctly determined as positive by the antigen combination, that is, the number of samples of patients with early Alzheimer's disease correctly determined as positive divided by the total number of samples of patients with early Alzheimer's disease; medium sensitivity refers to the proportion of 90 samples of patients with middle Alzheimer's disease correctly determined as positive, that is, in the middle stage, the number of positive samples from patients with Alzheimer's disease divided by the total number of samples from patients with mid-term Alzheimer's disease; late sensitivity refers to the proportion of 90 samples from patients with late Alzheimer's disease correctly determined to be positive, that is, the number of positive samples from patients with late Alzheimer's disease divided by the total number of samples from patients with late Alzheimer's disease. Overall sensitivity refers to the proportion of 180 samples of patients with early Alzheimer's disease, 90 samples of patients with intermediate Alzheimer's disease and 90 samples of patients with advanced Alzheimer's disease that are correctly determined as positive, that is, the number of samples of all patients with Alzheimer's disease divided by the total number of samples of all patients with Alzheimer's disease; overall specificity refers to the proportion of 180 samples of healthy subjects that are correctly determined as negative, that is, in 180 healthy subjects, the number of negative healthy subjects divided by the total number of healthy subjects.

The results of antigen combination test of each group are shown in Table 8.

**Table 8: Comparison of antigen combination test results of each group**

| group | Early sensitivity | Medium term sensitivity | Late sensitivity | Overall sensitivity | Overall specificity |
|---|---|---|---|---|---|
| Candidate group 1 | 94.44% | 88.89% | 93.33% | 92.78% | 90.00% |
| Candidate group 2 | 93.33% | 88.89% | 88.89% | 91.11% | 88.89% |
| Candidate group 3 | 94.44% | 84.44% | 91.11% | 91.11% | 88.33% |
| Candidate group 4 | 90.00% | 86.67% | 86.67% | 88.33% | 88.33% |
| Candidate group 5 | 92.22% | 88.89% | 91.11% | 91.11% | 90.00% |
| Candidate group 6 | 94.44% | 84.44% | 91.11% | 91.11% | 88.89% |
| Candidate group 7 | 93.33% | 91.11% | 80.00% | 89.44% | 90.00% |
| Candidate group 8 | 93.33% | 73.33% | 91.11% | 87.78% | 88.89% |
| Candidate group 9 | 74.44% | 88.89% | 91.11% | 82.22% | 88.33% |
| Candidate group 10 | 66.67% | 84.44% | 93.33% | 77.78% | 88.89% |
| Control group 1 | 77.78% | 66.67% | 80.00% | 75.56% | 83.33% |
| Control group 2 | 76.67% | 73.33% | 77.78% | 76.11% | 80.56% |
| Control group 3 | 80.00% | 62.22% | 80.00% | 75.56% | 77.78% |
| Control group 4 | 73.33% | 64.44% | 71.11% | 70.56% | 79.44% |
| Control group 5 | 75.56% | 66.67% | 75.56% | 73.33% | 80.00% |
| Control group 6 | 77.78% | 68.89% | 80.00% | 76.11% | 78.89% |
| Control group 7 | 78.89% | 68.89% | 77.78% | 76.11% | 77.78% |
| Control group 8 | 76.67% | 64.44% | 80.00% | 74.44% | 75.56% |
| Control group 9 | 83.33% | 77.78% | 82.22% | 81.67% | 87.22% |
| Control group 10 | 85.56% | 84.44% | 75.56% | 82.78% | 87.78% |

It can be seen from Table 8 that candidate groups 1-8 have higher sensitivity and specificity; the overall sensitivity is greater than 85%, and the overall specificity is greater than 88%. At the same time, compared with intermediate and late samples, the antigen combination provided by the invention has higher sensitivity in detecting early samples. Therefore, the invention provides a more accurate detection method for the diagnosis of Alzheimer's disease, especially for the early diagnosis.

In later application, one or more candidate antigen or antigen combinations provided by the invention can be selected as AD diagnostic kit according to needs.

## Claims

1. A protein antigen combination, comprising at least two protein fragments selected from the following proteins: MAPT, ADARB 1, P21, DNAJC8, RAGE, ASXL1, and JMJD2D.

2. The protein antigen combination according to claim 1, wherein at least one of the protein fragments is selected from the following sequences:
the MAPT protein fragment shown in SEQ ID No.1;
the ADARB1 protein fragment shown in SEQ ID No.2;
the P21 protein fragment shown in SEQ ID No.4;
the DNAJC8 protein fragment shown in SEQ ID No.6;
the RAGE protein fragment shown in SEQ ID No.7;
the ASXL1 protein fragment shown in SEQ ID No.8; and
the JMJD2D protein fragment shown in SEQ ID No.9.

3. The protein antigen combination according to claim 2, wherein the protein antigen combination further comprises one of the following proteins: HSP60 and DAG.

4. The protein antigen combination according to claim 3, wherein the HSP60 protein has the sequence shown in SEQ ID No.3; and the DAG protein has the sequence shown in SEQ ID No.5.

5. The protein antigen combination according to claim 4, comprising the MAPT protein fragment, the ADARB1 protein fragment and the HSP60 protein.

6. The protein antigen combination according to claim 4, comprising the DNAJC8 protein fragment, the RAGE protein fragment and the ASXL1 protein fragment.

7. The protein antigen combination according to claim 4, comprising the JMJD2D protein fragment, the P21 protein fragment and the DAG protein.

8. The protein antigen combination according to claim 4, comprising:
(i) the MAPT protein fragment, the HSP60 protein and the DNAJC8 protein fragment;
(ii) the ADARB 1 protein fragment, the RAGE protein fragment and the ASXL1 protein fragment; or
(iii) the HSP60 protein, the RAGE protein fragment and the P21 protein fragment.

9. A method of detecting Alzheimer's disease, comprising:
combining the protein antigen combination according to claim 1 with a biological sample from a subject at risk for Alzheimer's disease;
detecting autoantibodies against at least one of the protein fragments; and
predicting whether the subject has the risk of Alzheimer's disease, evaluating progress of Alzheimer's disease in the subject, or determining whether the subject has a risk of recurrence of Alzheimer's disease based on a result of detecting the autoantibodies.

10. A kit for detecting Alzheimer's disease, comprising:
the protein antigen combination of claim 1, purified and prepared for detecting autoantibodies against at least one of the protein fragments in a biological sample;
a labeled anti-human antibody that binds to the autoantibodies; and
a chromogenic agent that changes an absorbance of light at a predetermined wavelength in a test sample containing the protein antigen combination, the autoantibodies and the labeled anti-human antibody.

11. The kit according to claim 10, wherein the biological sample is whole blood or serum.

12. An antigen for detecting Alzheimer's disease, comprising a MAPT protein fragment having the sequence shown in SEQ ID No.1.

13. An antigen for detecting Alzheimer's disease, comprising a ADARB1 protein fragment having the sequence shown in SEQ ID No.2.

14. An antigen for detecting Alzheimer's disease, comprising a P21 protein fragment having the sequence shown in SEQ ID No.4.

15. An antigen for detecting Alzheimer's disease, comprising a DNAJC8 protein fragment having the sequence shown in SEQ ID No.6.

16. An antigen for detecting Alzheimer's disease, comprising a RAGE protein fragment having the sequence shown in SEQ ID No.7.

17. An antigen for detecting Alzheimer's disease, comprising a ASXL1 protein fragment having the sequence shown in SEQ ID No.8.

18. An antigen for detecting Alzheimer's disease, comprising a JMJD2D protein fragment having the sequence shown in SEQ ID No.9.
